# EUROPEAN PATENT SPECIFICATION

(11) **EP 2 421 573 B1**
(45) Date of publication and mention of the grant of the patent: **03.04.2013**
(21) Application number: 10716438.6
(22) Date of filing: 22.04.2010
(51) Int. Cl.: A61L 31/08, A61L 31/10, A61L 31/14, A61L 31/16

(54) **ENDOPROSTHESIS WITH SELECTIVE DRUG COATINGS**
ENDOPROTHESE MIT SELEKTIVEN ARZNEIMITTEL-ÜBERZÜGEN
ENDOPROTHÈSE AVEC DES REVÊTEMENTS DE MÉDICAMENT SÉLECTIFS

(30) Priority: 24.04.2009 US 429392
(43) Date of publication of application: 29.02.2012
(73) Proprietor: Boston Scientific Scimed, Inc., Maple Grove, MN 55311-1566 (US)
(72) Inventor: KUEHLING, Michael, 80804 Munich (DE); SCHEUERMANN, Torsten, 80803 Munich (DE); WEBER, Jan, NL-6228 GJ Maastricht (NL); XU, Yixin, Newton MA 02462 (US)
(74) Representative: Peterreins, Frank
(86) International application number: PCT/US2010/031990
(87) International publication number: WO 2010/124049

(56) References cited:
- WO-A1-2007/143433
- WO-A1-2009/089382
- WO-A2-2008/063539
- US-A1- 2006 129 215

## Description

### TECHNICAL FIELD

This disclosure relates to endoprostheses with a luminal surface, at least a portion of which is covered with a first coating including a first drug, and an abluminal surface, at least a portion of which is covered with a second coating including a second drug, wherein the first coating is formed of a bio-stable matrix material and the second coating is formed of a biodegradable matrix material.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, e.g., so that it can contact the walls of the lumen. Stent delivery is further discussed in Heath, U.S. 6,290,721.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn from the lumen.

Passageways containing endoprostheses can become re-occluded. Re-occlusion of such passageways is known as restenosis. It has been observed that certain drugs can inhibit the onset of restenosis when the drug is contained in the endoprosthesis. It is sometimes desirable for an endoprosthesis-contained therapeutic agent, or drug to elute into the body fluid in a predetermined manner once the endoprosthesis is implanted.

### SUMMARY

In an aspect, the invention features an endoprosthesis having a luminal surface, at least a portion of which is covered with a first coating including a first drug, and an abluminal surface, at least a portion of which is covered with a second coating including a second drug. The first drug has a first eluting profile based on the first coating, and the second drug has a second eluting profile based on the second coating, the first eluting profile being different from the second eluting profile. The first coating is formed of a bio-stable matrix material, and the second coating is formed of a biodegradable matrix material. The first and second coatings can be substantially free of polymer.

In another aspect, the invention features a method of forming an endoprosthesis that includes forming a first coating of a bio-stable material having a first drug on a luminal surface of the endoprosthesis, and forming a second coating of a biodegradable material having a second drug over a abluminal surface of the endoprosthesis. The first drug has a first eluting profile based on the first coating, and the second drug has a second eluting profile based on the second coating, the first eluting profile being different from the second eluting profile.

Embodiments may include one or more of the following features. The first coating has a thickness of about 200 nm or less. The first drug is embedded in the bio-stable matrix material. The first drug is an anti-coagulant: The anti-coagulant is heparin. The first drug and the bio-stable matrix material have a volume ratio of about 1 to 2 to about 3 to 2. The second drug is embedded in the biodegradable matrix material. The second drug is an anti-proliferative drug. The second drug is selected from paclitaxel, everolimus, tacrolimus, and sirolimus. The second drug and the biodegradable matrix material have a volume ratio of about 1 to 2 to about 3 to 2. The bio-stable matrix material has interconnected pores. The pores have a size of about 1-30 nm in diameter. The bio-stable matrix material is a metal, a metal nitride, or a metal oxide. The bio-stable metal is selected from silver, platinum, gold, and a mixture thereof. The bio-stable metal oxide or nitride is selected from oxide or nitride of iridium, zirconium, titanium, hafnium, niobium, tantalum, ruthenium, platinum, and a mixture thereof. The bio-stable metal oxide is iridium oxide. The bio-stable metal oxide or nitride has a smooth globular surface morphology to enhance endothelial cell growth on the oxide or the nitride. The biodegradable matrix material is a metal, or a metal salt. The biodegradable metal is selected from magnesium, calcium, aluminum, iron, zinc, titanium, and a mixture thereof. The biodegradable metal salt is selected from magnesium oxide, magnesium fluoride, tricalcium phosphate, calcium carbonate, and a mixture thereof. The biodegradable metal salt is tricalcium phosphate. The first coating is formed of a first bio-stable matrix material and the second coating is formed of a biodegradable material and a second bio-stable matrix material between the biodegradable material and the abluminal surface, where the first and the second bio-stable matrix materials are the same or different materials.

Embodiments may also include one or more of the following features. The bio-stable material and the first drug are co-deposited on the first region by pulsed laser deposition ("PLD"). The biodegradable material and the second drug are co-deposited on the second region by PLD. The bio-stable material is a metal, a metal nitride, or a metal oxide. The bio-stable metal oxide or nitride is selected from oxide or nitride of iridium, zirconium, titanium, hafnium, niobium, tantalum, ruthenium, platinum, and a mixture thereof. The bio-stable metal oxide is iridium oxide. The bio-stable metal oxide or nitride has a smooth globular surface morphology to enhance endothelial cell growth. The biodegradable material is a metal or a metal salt. The biodegradable metal salt is selected from magnesium oxide, magnesium fluoride, tricalcium phosphate, calcium carbonate, and a mixture thereof. The biodegradable metal salt is tricalcium phosphate. The first and second drugs are in the form of nano-sized particles. The nano-sized drug particles are coated by PLD using pneumatic fluidization.

Embodiments may include one or more of the following advantages. Stents can be formed with selective coating materials (e.g. as drug carriers) on select portions, such as the abluminal and luminal surfaces, to achieve different desirable functions on different portions of the stent. For example, it may be desirable that the luminal side or the blood-facing side of the stent be covered only by a thin layer of endothelial cells while the abluminal side or the tissue-facing side of the stent have a surface with low level of irritation or none to the surrounding tissues. Accordingly, the coating material overlying the luminal stent surface is formed of a bio-stable inorganic compound or ceramic, e.g., IROX, which has therapeutic advantages such as reducing the likelihood of restenosis and enhancing endothelial cell growth. The morphologies of the coating material can be selected to control the therapeutic properties of the coating and/or the porosity of the coating can be controlled to provide a desired drug release profile over an extended period. In comparison, the coating material overlying the abluminal stent surface is formed of a biodegradable inorganic compound, e.g., magnesium, tricalcium phosphate ("TCP"), or magnesium fluoride, which is capable of dissolving or being absorbed in a biological environment and thus capable of accelerating the release rate of a drug embedded in the coating material at the beginning after the stent implantation, for instance. The coating materials are substantially free of polymers, therefore avoiding any adverse effects caused by polymers such as acute or late thrombosis caused by polymer delamination. In addition, different drugs can be included in the abluminal and luminal coating materials to obtain predetermined therapeutic effects at different portions of the stent. For example, anti-proliferative drugs such as paclitaxel (PTX), Everolimus, and Tacrolimus, can be loaded in the abluminal coating material while anti-coagulants (e.g., Heparin) can be loaded in the luminal coating material. The coating materials and drugs can be selectively formed on the desired portions of the stent by low temperature deposition process, such as PLD, which reduce the likelihood of degradation of drugs by heat. Moreover, PLD enables the co-deposition of the coating materials and drugs and thus various drug-loading procedures after the formation of the drug carriers or reservoirs can be avoided. The coatings can also accommodate a large quantity of drugs and at the same time relatively thin.

Still further aspects, features, embodiments, and advantages follow.

### DESCRIPTION OF DRAWINGS

FIGS. 1A-1C are longitudinal cross-sectional views illustrating delivery of a stent in a collapsed state, expansion of the stent, and deployment of the stent.
FIG. 2 is a perspective view of a stent.
FIG. 3 is a cross-sectional schematic of a portion of a stent wall.
FIGS. 3A and 3B show different drug release profiles obtained at the abluminal and luminal sides of a stent by forming different coating materials on respective stent surfaces.
FIG. 4 is a cross-sectional schematic of drug elution.
FIG. 5 is a flow diagram illustrating manufacture of a stent.
FIG. 6 is a schematic of a PLD system.
FIGS. 7A-7C are FESEM images: FIG. 7A and 7B are enlarged plan views of a stent wall surface, FIG. 7C is an enlarged cross-sectional view of a stent wall surface.
FIGS. 8A-8C are schematics of ceramic morphologies..
FIG. 9 is a schematic of another embodiment of coating a stent.

### DETAILED DESCRIPTION

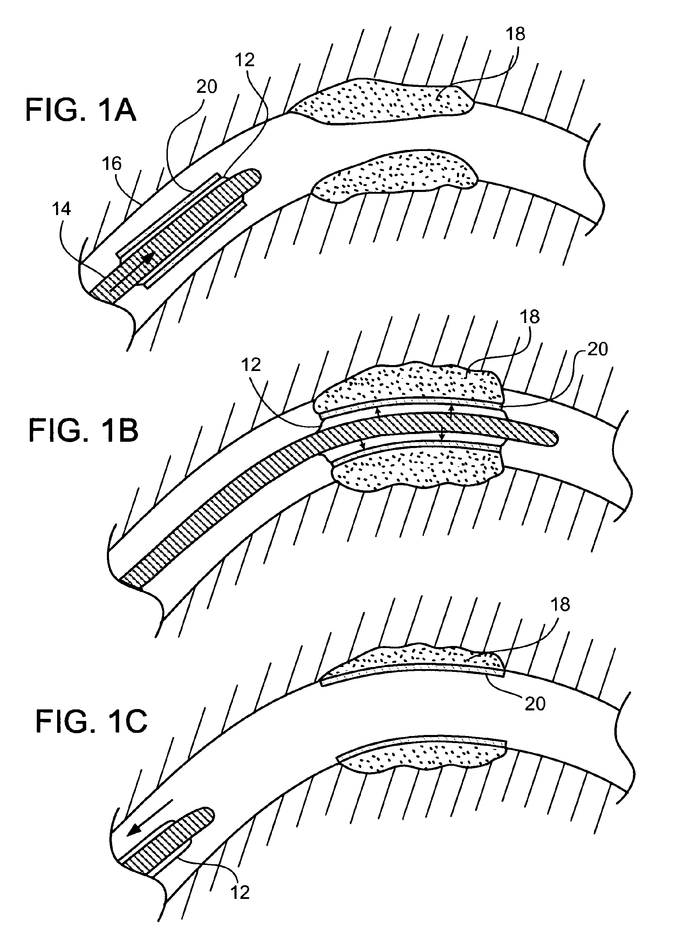

Referring to FIGS. 1A-1C, a stent 20 is placed over a balloon 12 carried near a distal end of a catheter 14, and is directed through the lumen 16 (FIG. 1A) until the portion carrying the balloon and stent reaches the region of an occlusion 18. The stent 20 is then radially expanded by inflating the balloon 12 and compressed against the vessel wall with the result that occlusion 18 is compressed, and the vessel wall surrounding it undergoes a radial expansion (FIG. 1B). The pressure is then released from the balloon and the catheter is withdrawn from the vessel (FIG. 1C).

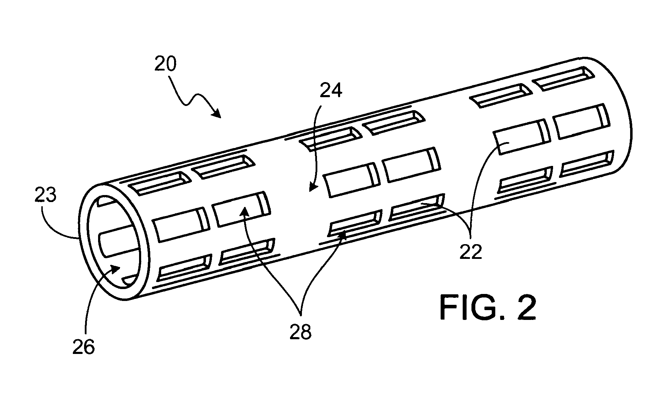

Referring to FIG. 2, the stent 20 includes a plurality of fenestrations 22 defined in a wall 23. Stent 20 includes several surface regions, including an outer, or abluminal, surface 24, an inner, luminal, surface 26, and a plurality of cutface surfaces 28. The stent can be balloon expandable, as illustrated above, or a self-expanding stent. Examples of stents are described in Heath `721, supra.

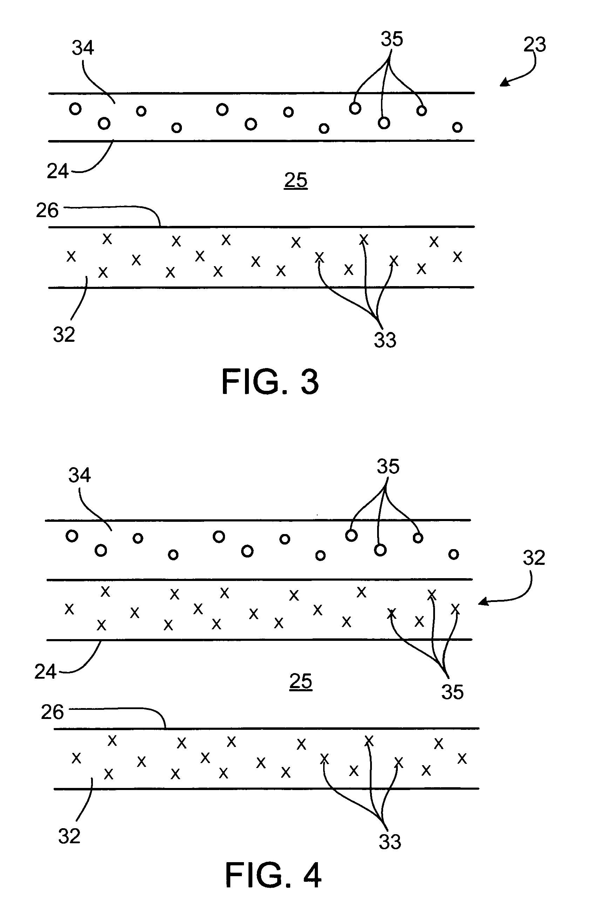

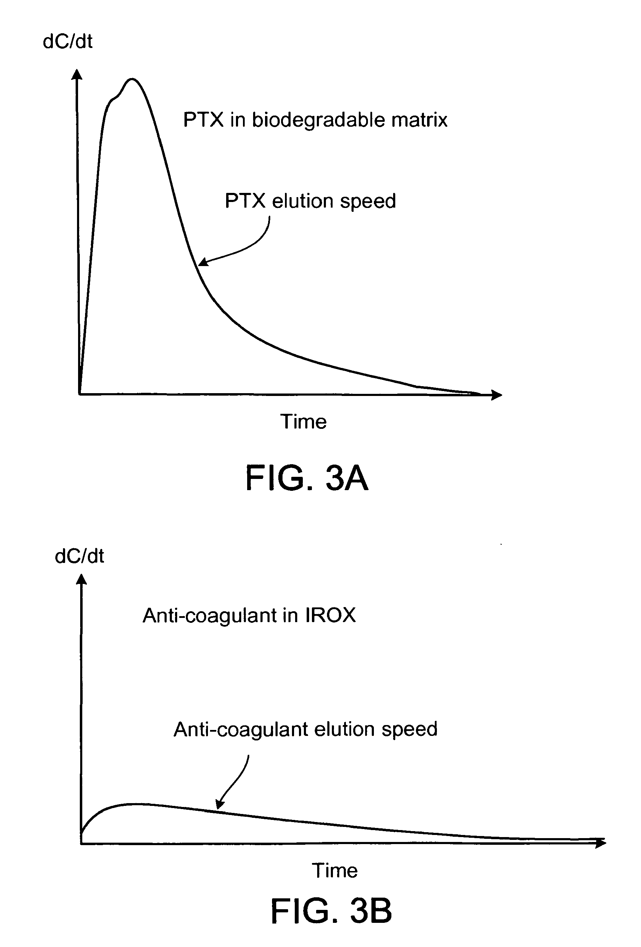

Referring to FIG. 3, a cross-sectional view, a stent wall 23 includes a stent body 25 formed, e.g. of a metal or a polymer; a luminal surface 26, at least part of which is covered by a first coating material 32, a bio-stable or permanent matrix, with a first drug 33 (illustrated as crosses) embedded therein; and an abluminal surface 24, at least part of which is covered by a second coating material 34, a biodegradable matrix, with a second drug 35 (illustrated as open circles) embedded therein. The first and second coating materials are different, e.g., their physical and/or chemical structures and properties in a biological environment can be different. The first and second drugs can be the same or different therapeutic agents.

In embodiments, the first coating and second coating materials are both non-polymeric materials such as inorganic compounds, e.g., metal, metal salts, metal oxides, nitrides, and halides, etc. The coating materials can also be selected from biological substances that primarily consist of inorganic compounds such as shells, bones, pearl, and corals.

The first coating material 32 is a bio-stable or permanent non-polymeric material, such as iridium oxide ("IROX"), which can have therapeutic advantages such as enhancing growth of a thin layer of endothelial cells as well as accommodating sufficient amounts of drug. The first drug 33 can be an anti-coagulant such as heparin. In embodiments, the bio-stable materials have porous structures, e.g., interconnected pores that can form channels for drug to elute into the surrounding biological environment. In embodiments, the first coating material or matrix with embedded first drug can form a layer on the luminal surface by a physical vapor deposition ("PVD") process. The layer has a thickness of about 10 nm to about 1000 nm, e.g., about 50-500nm, or about 100nm. In embodiments, the volume ratio of the drug and the matrix is about 1:2, or more, e.g. about 1:1 or more, e.g. about 3:2. In particular embodiment, the volume ratio of drug and matrix can vary at different depths of the coating, e.g., from 3:2 at an outermost surface of the coating to 1:2 at the coating/stent interface. In embodiments, the first coating material or the matrix has a pore size of about 1 to 30 nm. In particular embodiments, the bio-stable or permanent coating 32 are formed of an inorganic compound such as a metal (e.g., silver, platinum, iridium, zirconium, titanium, hafnium, niobium, tantalum or ruthenium, or gold), a metal oxide or a ceramic, such as iridium oxide ("IROX"), titanium oxide ("TiOₓ"), silicon oxide ("silica") or oxides of niobium ("Nb"), tantalum ("Ta"), ruthenium ("Ru") or mixture thereof. Without wishing to be bound by theory, it is believed that certain ceramics, e.g. oxides, can reduce restenosis, e.g., through the catalytic reduction of hydrogen peroxide and other precursors to smooth muscle cell proliferation, or their superior hemocompatibility. The oxides can also encourage endothelial growth to enhance endothelialization of the stent. When a stent is introduced into a biological environment (e.g., in vivo), one of the initial responses of the human body to the implantation of a stent, particularly into the blood vessels, is the activation of leukocytes, white blood cells which are one of the constituent elements of the circulating blood system. This activation causes an increase of reactive oxygen compound production. One of the species released in this process is hydrogen peroxide, H₂O₂, which is released by neutrophil granulocytes, which constitute one of the many types of leukocytes. The presence of H₂O₂ may increase proliferation of smooth muscle cells and compromise endothelial cell function, stimulating the expression of surface binding proteins which enhance the attachment of more inflammatory cells. A ceramic, such as IROX can catalytically reduce H₂O₂. The morphology of the ceramic can enhance the catalytic effect and reduce proliferation of smooth muscle cells. In a particular embodiment, IROX is selected to form the coating 32, which can have therapeutic benefits such as enhancing endothelialization. IROX and other ceramics are discussed further in Alt et al., U.S. Patent No. 5,980,566, USSN 10/651,562 filed August 29, 2003, and in Huang et al., Biomaterials 24 (2003): 2177-2187.

The second coating material 34 is a non-polymeric biodegradable material, such as a biodegradable metal, e.g., magnesium, iron, tungsten, or calcium, or a metal salt e.g., tricalcium phosphate (Ca₃(PO₄)₂ or TCP), calcium carbonate, biodegradable Hydroxyapatite (Ca₁₀(PO₄)₆(OH)₂ or HA) e.g., nanocrystalline HA, or magnesium fluoride, and the second drug 35 can be restenosis inhibitors or anti-proliferative drugs including paclitaxel ("PTX") or Everolimus. The bio-degradable coating can have either a porous structure or a nonporous structure. In embodiments, the second coating material or matrix with embedded second drug can form a layer on the abluminal surface by a physical vapor deposition ("PVD") process as well. The layer has a thickness of about 10 nm to about 1000 nm, e.g., about 50-500nm, or about 100nm. In embodiments, the volume ratio of the drug and the matrix is about 1:2, or more, e.g. about 1:1 or more, e.g. about 3:2. In particular embodiment, the volume ratio of drug and matrix can vary at different depths of the coating, e.g., from 1:2 at outmost surface of the coating to 3:2 at the coating/stent interface. Hydroxyapatite is discussed further in Kilian et al., Biomaterials 26 (2005): 1819-1827.

In embodiments, the discriminating selection of coating materials and/or drugs on different portions of the stent allows different desirable functions on different portions of the stent. For example, the luminal surface or the blood-facing side of the stent coated with the first coating material (e.g., IROX) can be covered only by a thin layer of endothelial cells while the abluminal surface or the tissue-facing side of the stent coated with the second coating material (e.g., TCP) can provide a surface with low level of irritation or none to the surrounding tissues. Moreover, in particular embodiments, different drug release profiles of different portions of the stent can also be achieved by selecting different coating materials. Referring particularly to FIGS. 3A and 3B, different drug release profiles at the abluminal and luminal sides of a stent can be obtained by forming different coating materials on respective stent surfaces. For example, FIG. 3A shows a predetermined PTX release profile in a biodegradable matrix at, e.g. the abluminal stent surface. The initial burst of PTX elution can be attributed to the biodegradable matrix erosion that leads to increasing exposure of PTX embedded in the matrix. FIG. 3B shows a different release profile of an anti-coagulant in an IROX matrix at, e.g. the luminal stent surface. In comparison, the relative steady release rate of a drug in a bio-stable matrix (e.g., IROX) can be attributed to the biostability of the matrix and its porous structure. In embodiments, drug release profile can also be selected by controlling the drug content or concentration at different depth of the matrix. For example, higher drug content at the outer surface of matrix will generate a release profile with an initial burst of drug release while higher drug content in a deeper layer of matrix will produce a release profile with a late burst.

In some embodiments, certain portions of the stent may have multiple coating materials, for example, the abluminal surface of the stent can have both the first and the second coating materials. Referring particularly to FIG. 4, both the abluminal and luminal surfaces are coated with the first material 32, e.g., IROX, and the first drug 33, e.g. an anti-coagulant, and only the abluminal surface has the second coating material 34, e.g., a non-polymeric biodegradable material, and the second drug 35, e.g., PTX, on top of the first coating. The first and the second drugs can be different or the same compounds. In still some embodiments, there may be multiple drugs in one coating matrix.

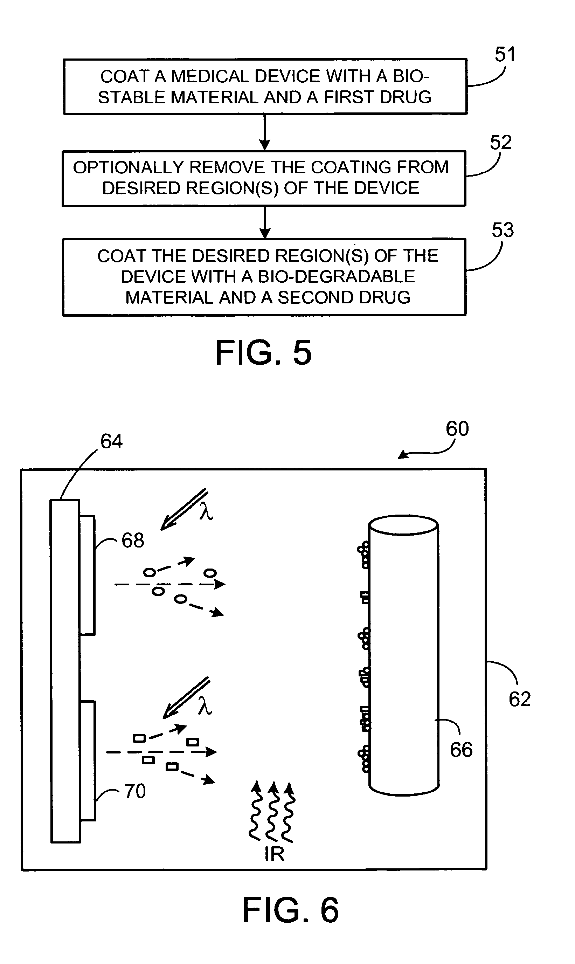

Referring to FIG. 5, a medical device, e.g., a stent is formed by first providing the first coating materialt, e.g. a bio-stable material, and a first drug on the device (step 51) by a technique that uses low temperature to reduce the likelihood of damaging the drug, such as pulsed layer deposition ("PLD"). Next, the coating of desired region(s) or surface(s) of the device can be optionally removed (step 52) by, e.g., laser ablation or argon ion milling processes. Finally, a second coating material, e.g., a biodegradable material, and a second drug are provided over the desired region(s) or surface(s) (step 53) by a technique that uses low temperature to reduce the likelihood of damaging the drug, such as PLD.

In a particular embodiment, all the surfaces of a stent (e.g., abluminal, luminal and cutface surfaces) are initially coated with a first coating material through co-deposition of IROX and Heparin particles without any mask. In a following step, a desired region, e.g., the luminal surface of the stent is masked or shielded by, e.g., a central metal stick or mandrel that supports the stent while the other surfaces are subjected to removal of the first coating via, e.g., ion milling, laser ablation or evaporation, so that the stent body can be revealed in those regions for a different coating. The stent, still masked on the same region, e.g., the luminal side, is then coated with a second coating material through co-deposition of a biodegradable matrix, e.g., TCP and PTX or Everolimus particles. The stent as produced will have a coating structure close to that illustrated in FIG. 3. In another particular embodiment, when the same procedure as described above has been followed except that no removing step is involved, the stent as produced will have a coating structure close to that illustrated in FIG. 4.

Referring particularly to steps 51 and 53 in FIG. 5, in embodiments, the first (or second) coating material or matrix and the first (or second) drug are provided over the stent surfaces by a technique that uses low temperature to reduce the likelihood of damaging the drug, such as PLD. One suitable class of PLD can be inverse PLD, which is discussed in Egerhazi et al., Applied Surface Science 247, 182-187 (2005). Referring to FIG. 6, the PLD system 60 includes a chamber 62 in which is provided a target assembly 64 and a stent substrate 66, such as a stent body or a pre-stent structure such as a metal tube. The target assembly includes a first target material 68, such as a bio-stable material (e.g., IROX), or a precursor to such a material (e.g., iridium metal), or a biodegradable material such as bio-erodible metal (e.g. magnesium) or salt (e.g. TCP), or naturally available biological substances such as shells, bones, pearl, and corals, and a second target material 70 such as a drug, e.g., an anti-coagulant or an anti-proliferative drug. Laser energy (double arrows) is selectively directed onto the target materials to cause the target materials to be ablated or sputtered from the target assembly. The sputtered material (often known as a laser-produced plasma plume, which is in the form of small clusters, e.g., neutrals, ions, electrons etc.) is imparted with kinetic energy in the ablation process such that the material is transported within the chamber (single arrows) and deposited on the stent 66. In addition, the temperature of the deposited material can be controlled by heating, e.g. using an infrared source (squiggly arrows).

The composition of the deposited materials (e.g., the relative abundance of the matrix and the drug) is selected by control of the deposition process. For example, the composition of the deposited materials is selected by controlling the exposure of the target materials to laser energy. To deposit pure matrix or pure drug, only the matrix or the drug target is exposed to laser energy. To deposit a composite layer of matrix and drug, both target materials are exposed simultaneously or alternately exposed in rapid succession. The relative amount of matrix material and drug is controlled by the laser energy and/or exposure time. For example, drug content in the matrix-drug composite layers can be controlled to vary gradually over the depth of the coating layers or to vary according to a predetermined release profile. In embodiments, the drug and matrix materials are deposited as small clusters, e.g., 200nm or less, such as 1-10nm, and preferably smaller than the gross morphological features of the layers.

In particular embodiments, the laser energy is produced by an excimer laser operating in the ultraviolet, e.g. at a wavelength of about 248 nm. The laser energy is about 100-700 mJ, the fluence is in the range of about 10 to 50 mJ/cm². The background pressure is in the range of about 1E-5 mbar to 1 mbar. The background gas includes oxygen. The substrate temperature is also controlled. The temperature of the substrate is between about 25 to about 300°C during deposition. In some embodiments, the temperature of the substrate is selected to be lower then the drug decomposition temperature, e.g., less than about 200°C, or even less than 100°C. Substrate temperature can be controlled by directing an infrared beam onto the substrate during deposition using, e.g. a halogen source. The temperature is measured by mounting a heat sensor in the beam adjacent the substrate. The temperature can be varied to control the morphology of the matrix material. The selective ablating of the matrix or drug is controlled by mounting the target materials on a moving assembly that can alternately bring the materials into the path of the laser. Alternatively, a beam splitter and shutter can be used to alternatively or simultaneously expose multiple materials. PLD deposition services are available from Axyntec (Augsburg, Germany). Suitable matrix materials include biostable or biodegradable ceramics, e.g. metal oxides and nitrides, such as of iridium, zirconium, titanium, hafnium, niobium, tantalum, ruthenium, platinum, calcium, magnesium, and aluminum. In embodiments, the thickness of the coatings is in the range of about 50 nm to about 2 um, e.g. 100 nm to 500 nm. Pulsed laser deposition is also described in application USSN 11/752,736, filed May 23, 2007 [Attorney Docket No. 10527-801001]. In other embodiments, another physical vapor deposition ("PVD") process is selected such as magnetron sputtering e.g. an iridium target under an oxygen atmosphere or an IROX target. Sputtering deposition is described in application USSN 11/752,772, filed May 23, 2007 [Attorney Docket No. 10527-805001].

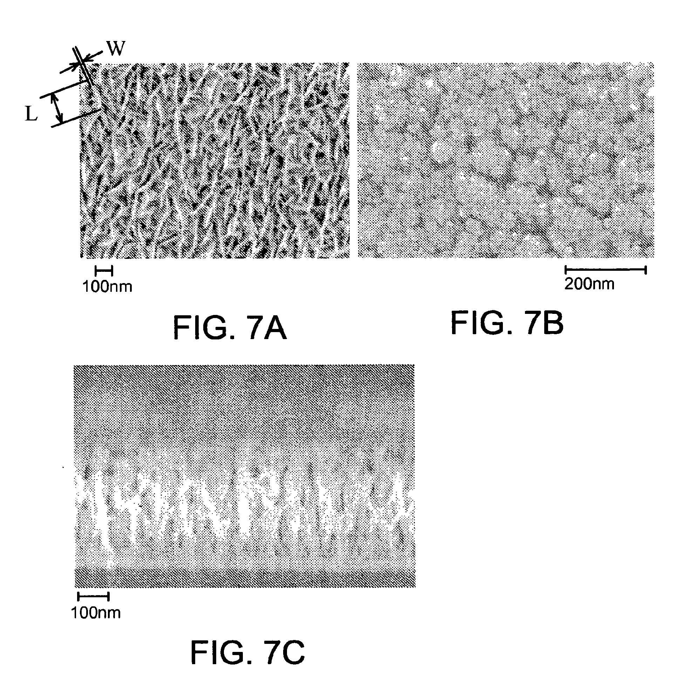

In particular embodiments, the bio-stable matrix coatings have surfaces with a select roughness or morphology as described in USSN 11/752,736, USSN 11/752,772, and appendices, supra. Referring to FIGS. 7A and 7B, the morphology of the bio-stable matrix such as a ceramic (e.g., IROX) can be varied between relatively rough surfaces and relatively smooth surfaces, which can each provide particular mechanical and therapeutic advantages, such as a controlled porosity to modulate drug release from the matrix. Referring particularly to FIG. 7A, a ceramic coating can have a morphology characterized by defined grains and high roughness. Referring particularly to FIG. 7B, a ceramic coating can have a morphology characterized by a higher coverage, globular surface of generally lower roughness. The defined grain, high roughness morphology provides a high surface area characterized by crevices between and around spaced grains into which, e.g., a polymer coating can be deposited and interlock to the surface, greatly enhancing adhesion. Defined grain morphologies also allow for greater freedom of motion and are less likely to fracture as the stent is flexed in use and thus the matrix coating resists delamination of the ceramic from an underlying surface and reduces delamination of a possible overlaying coating. The stresses caused by flexure of the stent during expansion or contraction of the stent or as the stent is delivered through a tortuously curved body lumen increase as a function of the distance from the stent axis. As a result, in embodiments, a morphology with defined grains is particularly desirable on abluminal regions of the stent or at other high stress points, such as the regions adjacent fenestrations which undergo greater flexure during expansion or contraction. Smoother globular surface morphology provides a surface which is tuned to facilitate endothelial growth by selection of its chemical composition and/or morphological features. Certain ceramics, e.g. oxides, can reduce restenosis through the catalytic reduction of hydrogen peroxide and other precursors to smooth muscle cell proliferation. The oxides can also encourage endothelial cell growth to enhance endothelialization of the stent. As discussed above, when a stent is introduced into a biological environment (e.g., in vivo), one of the initial responses of the human body to the implantation of a stent, particularly into the blood vessels, is the activation of white blood cells. This activation causes a release of hydrogen peroxide, H₂O₂. The presence of H₂O₂ may increase proliferation of smooth muscle cells and compromise endothelial cell function, stimulating the expression of surface binding proteins which enhance the attachment of more inflammatory cells. A ceramic, such as IROX can catalytically reduce H₂O₂. The smoother globular surface morphology of the ceramic can enhance the catalytic effect and enhance growth of endothelial cells.

Referring particularly to FIG. 7C, a cross-sectional view of the ceramic layer with surface morphology shown in FIG. 7B, channels formed of interconnected pores in the ceramic are visible. In embodiments, the channels have a diameter selectively controlled by deposition parameters as discussed above. The bigger the diameter, the faster the drug release rate. In particular embodiments, the channel diameter is selected to be about 10nm or less to control the drug release over a extended period of time.

The morphology of the ceramic is controlled by controlling the energy of the sputtered clusters on the stent substrate. Higher energies and higher temperatures result in defined grain, higher roughness surfaces. Higher energies are provided by increasing the temperature of the ceramic on the substrate, e.g. by heating the substrate or heating the ceramic with infrared radiation. In embodiments, defined grain morphologies are formed at temperatures of about 250°C or greater. Globular morphologies are formed at lower temperatures, e.g. ambient temperatures without external factors. The heating enhances the formation of a more crystalline ceramic, which forms the grains. Intermediate morphologies are formed at intermediate values of these parameters. The composition of the ceramic can also be varied. For example, oxygen content can be increased by providing oxygen gas in the chamber.

The morphology of the surface of the ceramic is characterized by its visual appearance, its roughness, and/or the size and arrangement of particular morphological features such as local maxima. In embodiments, the surface is characterized by definable sub-micron sized grains. Referring particularly to FIG. 7A, for example, in embodiments, the grains have a length, L, of about 50 to 500nm, e.g. about 100-300nm, and a width, W, of about 5nm to 50nm, e.g. about 10-15nm. The grains have an aspect ratio (length to width) of about 5:1 or more, e.g. 10:1 1 to 20:1. The grains overlap in one or more layers. The separation between grains can be about 1-50 nm. In particular embodiments, the grains resemble rice grains.

Referring particularly to FIG. 7B, in embodiments, the surface is characterized by a more continuous surface having a series of shallow globular features. The globular features are closely adjacent with a narrow minima between features. In embodiments, the surface resembles an orange peel. The diameter of the globular features is about 100nm or less, and the depth of the minima, or the height of the maxima of the globular function is, e.g., about 50nm or less, e.g. about 20nm or less. In other embodiments, the surface has characteristics between high aspect ratio definable grains and the more continuous globular surface and/or has a combination of these characteristics. For example, the morphology can include a substantially globular base layer and a relatively low density of defined grains. In other embodiments, the surface can include low aspect ratio, thin planar flakes. The morphology type is visible in FESEM images at 50 KX.

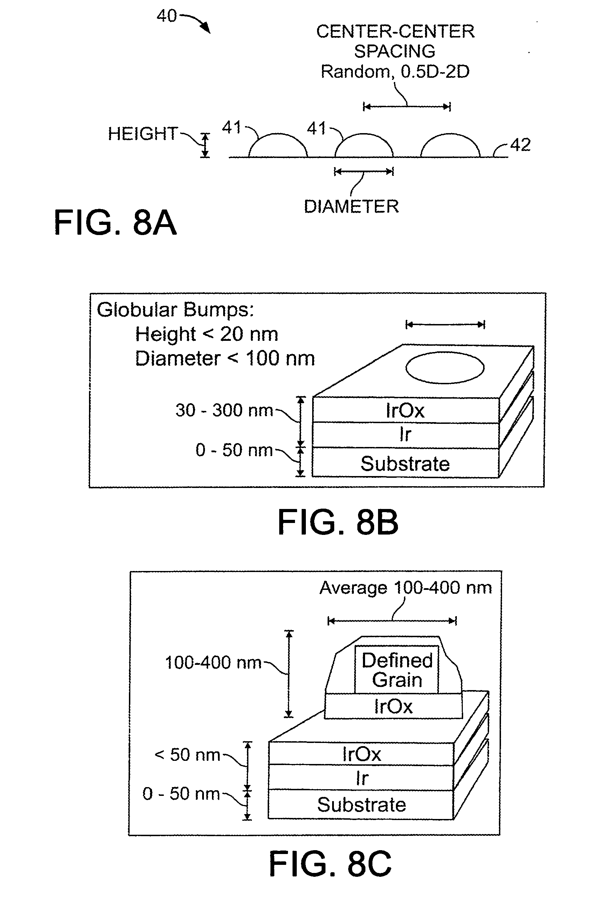

Referring to FIGS. 8A-8C, morphologies are also characterized by the size and arrangement of morphological features such as the spacing, height and width of local morphological maxima. Referring particularly to FIG. 8A, a coating 40 on a substrate 42 is characterized by the center-to-center distance and/or height, and/or diameter and/or density of local maxima. In particular embodiments, the average height, distance and diameter are in the range of about 400 nm or less, e.g. about 20-200 nm. In particular, the average center-to-center distance is about 0.5 to 2x the diameter.

Referring to FIG. 8B, in particular embodiments, the morphology type is a globular morphology, the width of local maxima is in the range of about 100nm or less and the peak height is about 20 nm or less. In particular embodiments, the ceramic has a peak height of less than about 5 nm, e.g., about 1-5 nm, and /or a peak distance less than about 15 nm, e.g., about 10-15 nm. Referring to FIG. 8C, in embodiments, the morphology is defined as a grain type morphology. The width of local maxima is about 400 nm or less, e.g. about 100-400 nm, and the height of local maxima is about 400 nm or less, e.g. about 100-400 nm. As illustrated in FIGS. 8B and 8C, the select morphologies of the ceramic can be formed on a thin layer of substantially uniform, generally amorphous IROX, which is in turn formed on a layer of iridium metal, which is in turn deposited on a metal substrate, such as titanium or stainless steel. The spacing, height and width parameters can be calculated from AFM data.

The roughness of the surface is characterized by the average roughness, Sa, the root mean square roughness, Sq, and/or the developed interfacial area ratio, Sdr. The Sa and Sq parameters represent an overall measure of the texture of the surface. Sa and Sq are relatively insensitive in differentiating peaks, valleys and the spacing of the various texture features. Surfaces with different visual morphologies can have similar Sa and Sq values. For a surface type, the Sa and Sq parameters indicate significant deviations in the texture characteristics. Sdr is expressed as the percentage of additional surface area contributed by the texture as compared to an ideal plane the size of the measurement region. Sdr further differentiates surfaces of similar amplitudes and average roughness. Typically Sdr will increase with the spatial intricacy of the texture whether or not Sa changes.

In embodiments, the ceramic has a defined grain type morphology. The Sdr is about 30 or more, e.g. about 40 to 60. In addition or in the alternative, the morphology has an Sq of about 15 or more, e.g. about 20 to 30. In embodiments, the Sdr is about 100 or more and the Sq is about 15 or more. In other embodiments, the ceramic has a globular type surface morphology. The Sdr is about 20 or less, e.g. about 8 to 15. The Sq is about 15 or less, e.g. about less than 8 to 14. In still other embodiments, the ceramic has a morphology between the defined grain and the globular surface, and Sdr and Sq values between the ranges above, e.g. an Sdr of about 1 to 200 and/or an Sq of about 1 to 30.

The morphology of the ceramic coating can exhibit high uniformity. The uniformity provides predictable, tuned therapeutic and mechanical performance of the ceramic. The uniformity of the morphology as characterized by Sa, Sq or Sdr and/or average peak spacing parameters can be within about +/- 20% or less, e.g. +/- 10% or less within a 1 µm square. In a given stent region, the uniformity is within about +/- 10%, e.g. about +/- 1 %. For example, in embodiments, the ceramic exhibits high uniformity over an entire surface region of stent, such as the entire abluminal or luminal surface, or a portion of a surface region, such as the center 25% or 50% of the surface region. The uniformity is expressed as standard deviation. Uniformity in a region of a stent can be determined by determining the average in five randomly chosen 1 µm square regions and calculating the standard deviation. Uniformity of a morphology type in a region is determined by inspection of FESEM data at 50 kx.

The ceramics are also characterized by surface composition, composition as a function of depth, and crystallinity. In particular, the amounts of oxygen or nitride in the ceramic is selected for a desired catalytic effect on, e.g., the reduction of H₂O₂ in biological processes. The composition of metal oxide or nitride ceramics can be determined as a ratio of the oxide or nitride to the base metal. In particular embodiments, the ratio is about 2 to 1 or greater, e.g. about 3 to 1 or greater, indicating high oxygen content of the surface. In other embodiments, the ratio is about 1 to 1 or less, e.g. about 1 to 2 or less, indicating a relatively low oxygen composition. In particular embodiments, low oxygen content globular morphologies are formed to enhance endothelialization. In other embodiments, high oxygen content defined grain morphologies are formed, e.g., to enhance adhesion and catalytic reduction. Composition can be determined by x-ray photoelectron spectroscopy (XPS). Depth studies are conducted by XPS after fast atomic beam ("FAB") sputtering. The crystalline nature of the ceramic can be characterized by crystal shapes as viewed in FESEM images, or Miller indices as determined by x-ray diffraction. In embodiments, defined grain morphologies have a Miller index of <101>. Globular materials have blended amorphous and crystalline phases that vary with oxygen content. Higher oxygen content typically indicates greater crystallinity.

In embodiments, the ceramic is deposited directly onto the metal surface of a stent body, e.g. a stainless steel, without the use of an intermediate metal layer. In other embodiments, a layer of metal common to the ceramic is deposited onto the stent body before deposition to the ceramic. For example, a layer of iridium may be deposited onto the stent body, followed by deposition of IROX onto the iridium layer. Other suitable ceramics, e.g., bio-stable ceramics, include metal oxides and nitrides, such as of iridium, zirconium, titanium, hafnium, niobium, tantalum, ruthenium, and platinum. The ceramic can be crystalline, partly crystalline or amorphous. The ceramic can be formed entirely of inorganic materials or a blend of inorganic and organic material (e.g. a polymer). In other embodiments, the morphologies described herein can be formed of metal.

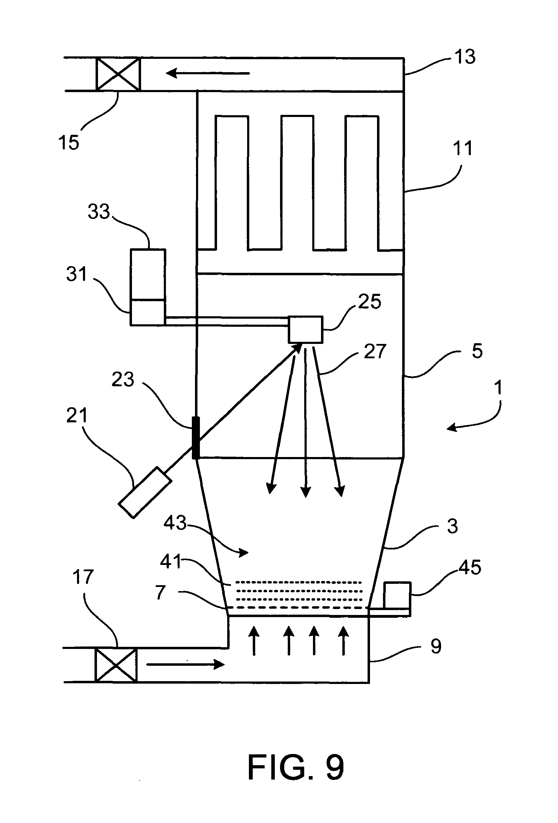

In another particular embodiment, the coating steps, e.g., steps 51 and 53 of FIG. 5 can use a PLD system as illustrated in FIG. 9. The PLD system allows coating solid nano-sized drug particles as a core material with a substance that is laser-ablated from a solid target material (polymer, oxide, etc.) under a pneumatic fluidization condition. In this embodiment, the drug particles are pre-formed before being placed in the PLD system while in the co-deposition process discussed above the drug clusters are formed in situ. The drug particles are about 1 micron or less at their largest dimension, e.g. 500nm or less. Suitable small particles, e.g. of paclitaxel, are available from Pharmasol GMBH (Blohmst 66 A, 12307 Berlin, Germany). Coating drug particles before depositing the drug onto a substrate, e.g. stent, may stabilize drug particles and further reduce the likelihood of drug damaging through PLD processes. A suitable PLD system is described in Talton, U.S. Pat. No. 7,063,748.

The drug coating material (i.e., material used to coat the drug particles) can be the matrix material, e.g., bio-stable or biodegradable inorganic compounds as discussed above. In other cases, the drug coating material can be a polymer. Suitable polymers include, for example, cellulose, polyacrylate, degradable polyester, copolymers with vinyl monomers such as isobutylene, isoprene and butadiene, for example, styrene-isobutylene-styrene (SIBS), styrene-isoprene-styrene (SIS) copolymers, styrenebutadiene-styrene (SBS) copolymers. Other suitable polymers, including biodegradable and non-biodegradable polymers, are discussed in U.S. Pat. No. 7,063,748 and USSN 11/752,736, filed May 23, 2007 [Attorney Docket No. 10527-801001]. The drug coating is on the order of from about 10 to about 1000 nm thick, preferably about 10 to 500 nm thick.

Referring particularly to FIG. 9, the apparatus is a top-coating apparatus 1 which includes a coating chamber 2, formed from a cylindrical portion 5 connected to a conical portion 3. Conical portion 3 is connected at its tapered end to a gas-permeable porous plate 7, and a gas distributor 9, adjacent to plate 7. At the opposite end of cylindrical portion 5, a filter 11 with cylindrical housing is mounted. An exhaust duct 13 carries gas for recirculation back through a filter assembly 15, through a blower (not shown), a temperature controller 17, then back to gas distributor 9 before re-entering the chamber. Top-coating apparatus 1 also includes an external evaporation or removal source (e.g., laser) 21, which is directed upward into central chamber 2 through window 23 to the matrix target (MT) 25, e.g., IROX, TCP, or SIBS, at approximately a 45 degree angle. Window 23 is formed from an optically transparent material, which is preferably quartz. The plume 27 leaves MT 25 downward toward the fluidized particles 41 below MT 25. Plume 27 coats onto core particles 41 which are contacted. The core particles 41, e.g., nano-sized drug particles, are preferably fluidized within the coating chamber to improve the uniformity of coating. An external control device 31 and container 33 are used to feed or turn MT 25, which may involve a rotating motor control and/or feeding tube. Container 33 may also include a chiller to freeze material for MT 25. A mechanical vibrator 45 can be used in conjunction with the gas fluidization to prevent particle agglomeration and to apply fluidization at lower gas flow regimes. The fluidization is preferably achieved by air/gas stream fluidization. That is, the core particles, e.g. drug particles, are placed in the path of flowing air or gas, which fluidizes the cores, improving their mixing and exposure to the coating chamber atmosphere.

Endoprostheses, e.g., stents, can be placed somewhere between the plume 27 and the plate 7, e.g., arrow 43, without blocking the fluidizing path or compromising coating of drug particles. The coated drug particles that are imparted with kinetic energies of the ablated matrix material as well as of fluidizing air/gas stream can then deposit on desired portions of the stent. The matrix material deposited on top of the coated drug can further secure the drug particles that coat the stent in place. In other embodiments, the coated drug particles can be deposited on the stent by applying optical forces , e.g., using optical tweezers, more detail of which is provided in Weber, US2005/0196614 A1, published September 8, 2005.

The terms "therapeutic agent", "pharmaceutically active agent", "pharmaceutically active material", "pharmaceutically active ingredient", "biologically active substance ", "drug" and other related terms may be used interchangeably herein and include, but are not limited to, small organic molecules, peptides, oligopeptides, proteins, nucleic acids, oligonucleotides, genetic therapeutic agents, non-genetic therapeutic agents, vectors for delivery of genetic therapeutic agents, cells, and therapeutic agents identified as candidates for vascular treatment regimens, for example, as agents that reduce or inhibit restenosis. By small organic molecule is meant an organic molecule having 50 or fewer carbon atoms, and fewer than 100 non-hydrogen atoms in total.

Exemplary therapeutic agents include, e.g., anti-thrombogenic agents (e.g., heparin); anti-proliferative/anti-mitotic agents (e.g., paclitaxel, 5-fluorouracil, cisplatin, vinblastine, vincristine, inhibitors of smooth muscle cell proliferation (e.g., monoclonal antibodies), and thymidine kinase inhibitors); antioxidants; anti-inflammatory agents (e.g., dexamethasone, prednisolone, corticosterone); anesthetic agents (e.g., lidocaine, bupivacaine and ropivacaine); anti-coagulants; antibiotics (e.g., erythromycin, triclosan, cephalosporins, and aminoglycosides); agents that stimulate endothelial cell growth and/or attachment. Therapeutic agents can be nonionic, or they can be anionic and/or cationic in nature. Therapeutic agents can be used singularly, or in combination. Preferred therapeutic agents include inhibitors of restenosis (e.g., paclitaxel), immunosuppressants(e.g., everolimus, tacrolimus, Sirolimus), anti-proliferative agents (e.g., cisplatin), and antibiotics (e.g., erythromycin). Additional examples of therapeutic agents are described in U.S. Published Patent Application No. 2005/0216074. Polymers for drug elution coatings are also disclosed in U.S. Published Patent Application Nos. 2005/0019265 and 2005/0251249. A functional molecule, e.g. an organic, drug, polymer, protein, DNA, and similar material can be incorporated into groves, pits, void spaces, and other features of the ceramic.

Any stent described herein can be dyed or rendered radiopaque by addition of, e.g., radiopaque materials such as barium sulfate, platinum or gold, or by coating with a radiopaque material. The stent can include (e.g., be manufactured from) metallic materials, such as stainless steel (e.g., 316L, BioDur® 108 (UNS S29108), and 304L stainless steel, and an alloy including stainless steel and 5-60% by weight of one or more radiopaque elements (e.g., Pt, Ir, Au, W) (PERKS®) as described in US2003/0018380 A1, US2002/0144757 A1, and US2003/0077200 A1), Nitinol (a nickel-titanium alloy), cobalt alloys such as Elgiloy, L605 alloys, MP35N, titanium, titanium alloys (e.g., Ti-6A1-4V, Ti-50Ta, Ti-10Ir), platinum, platinum alloys, niobium, niobium alloys (e.g., Nb-1Zr) Co-28Cr-6Mo, tantalum, and tantalum alloys. Other examples of materials are described in commonly assigned U.S. Application No. 10/672,891, filed September 26, 2003; and U.S. Application No. 11/035,316, filed January 3, 2005. Other materials include elastic biocompatible metal such as a superelastic or pseudo-elastic metal alloy, as described, for example, in Schetsky, L. McDonald, "Shape Memory Alloys", Encyclopedia of Chemical Technology (3rd ed.), John Wiley & Sons, 1982, vol. 20. pp. 726-736; and commonly assigned U.S. Application No. 10/346,487, filed January 17, 2003.

The stents described herein can be configured for vascular, e.g. coronary and peripheral vasculature or non-vascular lumens. For example, they can be configured for use in the esophagus or the prostate. Other lumens include biliary lumens, hepatic lumens, pancreatic lumens, urethral lumens.

The stent can be of a desired shape and size (e.g., coronary stents, aortic stents, peripheral vascular stents, gastrointestinal stents, urology stents, tracheal/bronchial stents, and neurology stents). Depending on the application, the stent can have a diameter of between, e.g., about 1 mm to about 46 mm. In certain embodiments, a coronary stent can have an expanded diameter of from about 2 mm to about 6 mm. In some embodiments, a peripheral stent can have an expanded diameter of from about 4 mm to about 24 mm. In certain embodiments, a gastrointestinal and/or urology stent can have an expanded diameter of from about 6 mm to about 30 mm. In some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm. The stent can be balloon-expandable, self-expandable, or a combination of both (e.g., U.S. Patent No. 6,290,721). The discriminating selection of different coatings and/or different drugs on different regions, e.g., bio-stable coating on the blood-facing region and biodegradable coating on the tissue-facing region, can be applied to other endoprostheses or medical devices, such as catheters, guide wires, valves, and filters.

## Claims

1. An endoprosthesis, comprising:
a luminal surface, at least a portion of which is covered with a first coating including a first drug, the first drug having a first eluting profile based on the first coating, and
an abluminal surface, at least a portion of which is covered with a second coating including a second drug, the second drug having a second eluting profile based on the second coating, the first eluting profile being different from the second eluting profile, wherein the first coating is formed of a bio-stable matrix material and wherein the second coating is formed of a biodegradable matrix material.

2. The endoprosthesis of claim 1, wherein the first and second coatings are free of polymer.

3. The endoprosthesis of claim 1, wherein the first coating has a thickness of 200 nm or less.

4. The endoprosthesis of claim 1, wherein the first drug is embedded in the bio- stable matrix material.

5. The endoprosthesis of claim 4, wherein the first drug is an anti-coagulant such as heparin.

6. The endoprosthesis of claim 1, wherein the second drug is embedded in the biodegradable matrix material.

7. The endoprosthesis of claim 6, wherein the second drug is an anti-proliferative drug selected from paclitaxel, everolimus, tacrolimus, and sirolimus.

8. The endoprosthesis of claim 1, wherein the bio-stable matrix material has interconnected pores.

9. The endoprosthesis of claim 8, wherein the bio-stable matrix material is a metal, a metal nitride, or a metal oxide, the metal being selected from silver, platinum, gold, iridium, zirconium, titanium, hafnium, niobium, tantalum, ruthenium, and a mixture thereof, and the oxide or nitride being selected from oxide or nitride of iridium, zirconium, titanium, hafnium, niobium, tantalum, ruthenium, platinum, and a mixture thereof

10. The endoprosthesis of claim 9, wherein the oxide or nitride has a smooth globular surface morphology to enhance endothelial cell growth on the oxide or the nitride.

11. The endoprosthesis of claim 1, wherein the biodegradable matrix material is a metal, or a metal salt, the metal being selected from magnesium, calcium, aluminum, iron, zinc, titanium, and a mixture thereof, and the metal salt being selected from magnesium oxide, magnesium fluoride, tricalcium phosphate, calcium carbonate, and a mixture thereof

12. The endoprosthesis of claim 1. wherein the first coating is formed of a first bio-stable matrix material and the second coating is formed of a biodegradable material and a second bio-stable matrix material between the biodegradable material and the ab luminal surface, wherein the first and the second bio-stable matrix materials are the same or different materials.

13. A method of forming an endoprosthesis, comprising:
forming a first coating of a bio-stable material having a first drug on a luminal surface of the endoprosthesis so that the first drug has a first eluting profile based on the first coating, and
forming a second coating of a biodegradable material having a second drug on an abluminal surface of the endoprosthesis so that the second drug has a second eluting profile based on the second coating, the first eluting profile being different from the second eluting profile.

14. The method of claim 13, wherein the bio-stable material and the first drug are co-deposited on the first region by PLD, or wherein the biodegradable material and the second drug are co-deposited on the second region by PLD.

15. The method of claim 13, wherein the bio-stable material is a metal, a metal nitride, or a metal oxide, the metal oxide or nitride being selected from oxide or nitride of iridium, zirconium, titanium, hafnium, niobium, tantalum, ruthenium, platinum, and a mixture thereof.

## Patentansprüche

1. Endoprothese, umfassend:
eine luminale Oberfläche, wobei mindestens ein Teil dieser mit einer ersten Beschichtung bedeckt ist, die einen ersten Wirkstoff beinhaltet, wobei der erste Wirkstoff ein erstes Elutionsprofil hat, basierend auf der ersten Beschichtung, und
eine abluminale Oberfläche, wobei mindestens ein Teil dieser mit einer zweiten Beschichtung bedeckt ist, die einen zweiten Wirkstoff beinhaltet,
wobei der zweite Wirkstoff ein zweites Elutionsprofil hat, basierend auf der zweiten Beschichtung, wobei das erste Elutionsprofil unterschiedlich zu dem zweiten Elutionsprofil ist, wobei die erste Beschichtung aus einem biostabilen Matrixmaterial gebildet ist und wobei die zweite Beschichtung aus einem bioabbaubaren Matrixmaterial gebildet ist.

2. Endoprothese nach Anspruch 1, wobei die ersten und zweiten Beschichtungen frei von Polymer sind.

3. Endoprothese nach Anspruch 1, wobei die erste Beschichtung eine Dicke von 200 nm oder weniger hat.

4. Endoprothese nach Anspruch 1, wobei der erste Wirkstoff in das biostabile Matrixmaterial eingebettet ist.

5. Endoprothese nach Anspruch 4, wobei der erste Wirkstoff ein Antikoagulans wie zum Beispiel Heparin ist.

6. Endoprothese nach Anspruch 1, wobei der zweite Wirkstoff in das bioabbaubaren Matrixmaterial eingebettet ist.

7. Endoprothese nach Anspruch 6, wobei der zweite Wirkstoff ein antiproliferativer Wirkstoff ist, ausgewählt aus Paclitaxel, Everolimus, Tacrolimus und Sirolimus.

8. Endoprothese nach Anspruch 1, wobei das biostabile Matrixmaterial miteinander verbundene Poren hat.

9. Endoprothese nach Anspruch 8, wobei das biostabile Matrixmaterial ein Metall, ein Metallnitrid oder ein Metalloxid ist, wobei das Metall ausgewählt ist aus Silber, Platin, Gold, Iridium, Zirkon, Titan, Hafnium, Niob, Tantal, Ruthenium und einem Gemisch daraus, und das Oxid oder Nitrid ausgewählt ist aus Oxid oder Nitrid von Iridium, Zirkon, Titan, Hafnium, Niob, Tantal, Ruthenium, Platin und einem Gemisch daraus.

10. Endoprothese nach Anspruch 9, wobei das Oxid oder Nitrid eine glatte kugelförmige Oberflächenmorphologie hat, um den endothelialen Zellwachstum auf dem Oxid oder Nitrid zu steigern.

11. Endoprothese nach Anspruch 1, wobei das bioabbaubare Matrixmaterial ein Metall oder ein Metallsalz ist, wobei das Metall ausgewählt ist aus Magnesium, Calcium, Eisen, Zink, Titan, und einem Gemisch daraus, und das Metallsalz ausgewählt ist aus Magnesiumoxid, Magnesiumfluorid, Tricalciumphosphat, Calciumcarbonat und einem Gemisch daraus.

12. Endoprothese nach Anspruch 1, wobei die erste Beschichtung aus einem ersten biostabilen Matrixmaterial gebildet ist und die zweite Beschichtung aus einem bioabbaubaren Material und einem zweiten biostabilen Matrixmaterial gebildet ist, zwischen dem bioabbaubaren Material und der abluminalen Oberfläche, wobei die ersten und die zweiten biostabilen Matrixmaterialien die gleichen oder verschiedene Materialien sind.

13. Verfahren zum Bilden einer Endoprothese, umfassend:
Bilden einer ersten Beschichtung aus einem biostabilen Material mit einem ersten Wirkstoff auf einer luminalen Oberfläche der Endoprothese, so dass der erste Wirkstoff ein erstes Elutionsprofil hat, basierend auf der ersten Beschichtung, und
Bilden einer zweiten Beschichtung aus einem bioabbaubaren Material mit einem zweiten Wirkstoff auf einer abluminalen Oberfläche der Endoprothese, so dass der zweite Wirkstoff ein zweites Elutionsprofil hat, basierend auf der zweiten Beschichtung, wobei das erste Elutionsprofil unterschiedlich zu dem zweiten Elutionsprofil ist.

14. Verfahren nach Anspruch 13, wobei das biostabile Material und der erste Wirkstoff gleichzeitig in der ersten Region mittels PLD abgeschieden werden, oder wobei das bioabbaubare Material und der zweite Wirkstoff gleichzeitig in der zweiten Region mittels PLD abgeschieden werden.

15. Verfahren nach Anspruch 13, wobei das biostabile Material ein Metall, ein Metallnitrid oder ein Metalloxid ist, wobei das Metalloxid oder -nitrid ausgewählt ist aus Oxid oder Nitrid von Iridium, Zirkon, Titan, Hafnium, Niob, Tantal, Ruthenium, Platin und ein Gemisch daraus.

## Revendications

1. Endoprothèse, comprenant :
une surface luminale dont au moins une partie est recouverte par un premier revêtement comprenant un premier médicament, le premier médicament présentant un premier profil d'élution basé sur le premier revêtement, et
une surface abluminale dont au moins une partie est recouverte par un deuxième revêtement comprenant un deuxième médicament, le deuxième médicament présentant un deuxième profil d'élution basé sur le deuxième revêtement, le premier profil d'élution étant différent du deuxième profil d'élution, le premier revêtement étant constitué d'un matériau de matrice biostable et le deuxième revêtement étant constitué d'un matériau de matrice biodégradable.

2. Endoprothèse selon la revendication 1, dans laquelle le premier et le deuxième revêtement sont dépourvus de polymère.

3. Endoprothèse selon la revendication 1, dans laquelle le premier revêtement a une épaisseur de 200 nm ou moins.

4. Endoprothèse selon la revendication 1, dans laquelle le premier médicament est incorporé dans le matériau de matrice biostable.

5. Endoprothèse selon la revendication 4, dans laquelle le premier médicament est un anticoagulant tel que l'héparine.

6. Endoprothèse selon la revendication 1, dans laquelle le deuxième médicament est incorporé dans le matériau de matrice biodégradable.

7. Endoprothèse selon la revendication 6, dans laquelle le deuxième médicament est un médicament antiprolifératif choisi parmi le paclitaxel, l'évérolimus, le tacrolimus et le sirolimus.

8. Endoprothèse selon la revendication 1, dans laquelle le matériau de matrice biostable comporte des pores interconnectés.

9. Endoprothèse selon la revendication 8, dans laquelle le matériau de matrice biostable est un métal, un nitrure métallique ou un oxyde métallique, le métal étant choisi parmi l'argent, le platine, l'or, l'iridium, le zirconium, le titane, le hafnium, le niobium, le tantale, le ruthénium, et un mélange de ceux-ci, et l'oxyde ou le nitrure étant choisi parmi l'oxyde ou le nitrure d'iridium, de zirconium, de titane, de hafnium, de niobium, de tantale, de ruthénium, de platine, et d'un mélange de ceux-ci.

10. Endoprothèse selon la revendication 9, dans laquelle l'oxyde ou le nitrure a une morphologie de surface globulaire lisse pour améliorer la croissance des cellules endothéliales sur l'oxyde ou le nitrure.

11. Endoprothèse selon la revendication 1, dans laquelle le matériau de matrice biodégradable est un métal ou un sel métallique, le métal étant choisi parmi le magnésium, le calcium, l'aluminium, le fer, le zinc, le titane, et un mélange de ceux-ci, et le sel métallique étant choisi parmi l'oxyde de magnésium, le fluorure de magnésium, le phosphate tricalcique, le carbonate de calcium, et un mélange de ceux-ci.

12. Endoprothèse selon la revendication 1, dans laquelle le premier revêtement est constitué d'un premier matériau de matrice biostable et le deuxième revêtement est constitué d'un matériau biodégradable et d'un deuxième matériau de matrice biostable entre le matériau biodégradable et la surface abluminale, le premier et le deuxième matériau de matrice biostable étant des matériaux identiques ou différents.

13. Procédé de formation d'une endoprothèse, comprenant :
la formation d'un premier revêtement d'un matériau biostable comprenant un premier médicament sur une surface luminale de l'endoprothèse de telle sorte que le premier médicament présente un premier profil d'élution basé sur le premier revêtement, et
la formation d'un deuxième revêtement d'un matériau biodégradable comprenant un deuxième médicament sur une surface abluminale de l'endoprothèse de telle sorte que le deuxième médicament présente un deuxième profil d'élution basé sur le deuxième revêtement, le premier profil d'élution étant différent du deuxième profil d'élution.

14. Procédé selon la revendication 13, dans lequel le matériau biostable et le premier médicament sont codéposés sur la première région par PLD, ou dans lequel le matériau biodégradable et le deuxième médicament sont codéposés sur la deuxième région par PLD.

15. Procédé selon la revendication 13, dans lequel le matériau biostable est un métal, un nitrure métallique ou un oxyde métallique, l'oxyde ou le nitrure métallique étant choisi parmi l'oxyde ou le nitrure d'iridium, de zirconium, de titane, de hafnium, de niobium, de tantale, de ruthénium, de platine, et d'un mélange de ceux-ci.
